# EUROPEAN PATENT SPECIFICATION

(11) **EP 3 130 337 B1**
(45) Date of publication and mention of the grant of the patent: **27.03.2019**
(21) Application number: 15717941.7
(22) Date of filing: 31.03.2015
(51) Int. Cl.: A61K 31/191, A61K 31/194, A61K 31/704, A61K 36/258, A61K 36/484, A61K 36/77, A23L 33/105, A61K 45/06, A61P 13/02, A61P 13/04

(54) **COMBINATION OF A URINARY BASIFYING AGENT AND AN URIC ACID CRYSTALLISATION INHIBITOR FOR THE TREATMENT OR PREVENTION OF RENAL LITHIASIS**
KOMBINATION EINES HARNALKALISIERUNSMITTELS UND EINES HARNSÄUREKRISTALLISATIONSHEMMERS ZUR VORBEUGUNG ODER BEHANDLUNG VON NIERENLITHIASIS
COMBINAISON D'UN AGENT ALCALINISANT URINAIRE ET D'UN INHIBITEUR DE LA CRYSTALLISATION DE L'ACIDE URIQUE POUR LE TRAITEMENT OU LA PRÉVENTION DE LA LITHIASE RÉNALE

(30) Priority: 02.04.2014 ES 201430480
(43) Date of publication of application: 15.02.2017
(73) Proprietor: Universitat de les Illes Balears, 07120 Palma de Mallorca (Islas Baleares) (ES)
(72) Inventor: GRASES FREIXEDAS, Félix, E-07120 Palma de Mallorca (Islas Baleares) (ES); COSTA BAUZÁ, Antonia, E-07120 Palma de Mallorca (Islas Baleares) (ES); PRIETO ALMIRALL, Rafael María, E-07120 Palma de Mallorca (Islas Baleares) (ES); RODRÍGUEZ RODRÍGUEZ, Adrían, E-07120 Palma de Mallorca (Islas Baleares) (ES)
(74) Representative: ZBM Patents ApS
(86) International application number: PCT/ES2015/070250
(87) International publication number: WO 2015/150609

(56) References cited:
- GB-A- 2 406 853
- US-A- 3 238 190
- US-A1- 2002 090 445
- DATABASE WPI Week 201321 Thomson Scientific, London, GB; AN 2013-B97005 XP002740657, & CN 102 764 420 A (WANG L) 7 November 2012 (2012-11-07)
- DATABASE WPI Week 201030 Thomson Scientific, London, GB; AN 2010-E30514 XP002740658, & CN 101 690 740 A (LIU S) 7 April 2010 (2010-04-07)
- GRASES F ET AL: "Uric acid urolithiasis and crystallization inhibitors", UROLOGIA INTERNATIONALIS, KARGER, CH, vol. 62, no. 4, 1 November 1999 (1999-11-01), pages 201-204, XP008176575, ISSN: 0042-1138, DOI: 10.1159/000030395 [retrieved on 1999-12-11]
- HISATOME ICHIRO ET AL: "Renal hypouricemia due to enhanced tubular secretion of urate associated with urolithiasis: Successful treatment of urolithiasis by alkalization of urine K+, Na+-citrate", NEPHRON, S. KARGER AG, SWITZERLAND, vol. 65, no. 4, 1 January 1993 (1993-01-01), pages 578-582, XP008176588, ISSN: 0028-2766, DOI: 10.1159/000187567 [retrieved on 2008-12-12]
- GRASES FELIX ET AL: "Renal lithiasis and nutrition", NUTRITION JOURNAL, BIOMED CENTRAL, GB, vol. 5, no. 1, 6 September 2006 (2006-09-06), page 23, XP021017998, ISSN: 1475-2891, DOI: 10.1186/1475-2891-5-23
- HEILBERG ITA P ET AL: "Optimum nutrition for kidneystone disease", ADVANCES IN CHRONIC KIDNEY DISEASE JUL 2008, SAUNDERS, US, vol. 20, no. 2, 1 March 2013 (2013-03-01), pages 165-174, XP008176573, ISSN: 1548-5609, DOI: 10.1053/J.ACKD.2012.12.001 [retrieved on 2013-02-21]
- GRASES F ET AL: "STUDY OF THE EFFECTS OF DIFFERENT SUBSTANCES ON THE EARLY STAGES OF PAPILLARY STONE FORMATION", NEPHRON, S. KARGER AG, SWITZERLAND, vol. 73, no. 4, 1 August 1996 (1996-08-01) , pages 561-568, XP000617412, ISSN: 0028-2766
- DATABASE WPI Week 201378 Thomson Scientific, London, GB; AN 2013-S49003 XP002740659, & CN 103 169 767 A (MAYINGLONG PHARM GROUP CO LTD WUHAN) 26 June 2013 (2013-06-26)

## Description

The present invention relates to the use of a separate, sequential or simultaneous combination of at least one urinary basifying agent, with at least one uric acid crystallization inhibitor for basifying urine as defined in the claims; thus avoiding the pH value at which uric acid becomes oversaturated and, as a result, renal lithiasis.

### STATE OF THE ART

The oral consumption of substances such as citrate, polyhydroxycarboxylic acids, sodium and potassium bicarbonates, etc., is known to increase urinary pH values. This is because, given their metabolism, these substances increase the reabsorption of hydrogenions at nephronal level, such that the urine is basified as a result. Moreover, the fundamental problem of uric renal lithiasis is now known to reside in the more or less persistent existence of urinary pH values of below 5.5. Although the oversaturation of uric acid in urine, in addition to the excretion of this substance is important, practically no cases demonstrate that is a decisive factor in the development of lithiasis. The presence of uric acid crystallization inhibitors is believed to be relevant, since certain individuals with the same urinary pH values and uric acid concentrations form uric renal calculi, whilst others do not. However, despite the fact that several "in vitro" studies have been carried out on potential uric acid crystallization inhibitors (Grases F, Ramis M, Villacampa Al, Costa-Bauzá A. Uric acid urolithiasis and crystallization inhibitors. Urol Int. 1999;62:201-4), in which saponins, glycosaminoglycans and glycoproteins have been shown to exhibit a remarkable ability to inhibit uric acid crystallization , no clinical studies on this matter are known.
At present, the prophylactic treatment of uric acid renal lithiasis is based on the implementation of dietary measures designed to decrease uric acid levels in urine and in turn, increase urinary pH levels. As such, decreasing excessive consumption of animal origin proteins (red meat, seafood, oily fish and viscera, etc.) and alcoholic drinks is recommended, as well as increasing consumption of fruits (especially citrus fruits) and vegetables, in addition to carbonated drinks. The only drug currently used to treat uric renal lithiasis is citrate. In some cases, urinary basifying agents may even be used to re-dissolve uric acid calculi, when the size and location thereof is appropriate. In addition to being linked to the generation of uric acid calculi, the presence of uric acid crystals in urine may give rise to the development of (apparently "pure") calcium oxalate calculi through heterogeneous nucleation mechanisms, and the formation of mixed calcium oxalate/uric acid calculi, which has increased significantly over the past few years, given that, despite citrate treatment proving highly effective in decreasing the formation of this kind of calculi, the same nevertheless tend to have a high relapse rate. The only limitation of uncontrolled use of high doses of citrate resides in the fact that if urinary pH rises to values of above 6.2, urine becomes oversaturated in calcium phosphate, and the formation of phosphate or even calcium phosphate lithiasis may induce the formation of apparently pure calcium oxalate calculi by means of heterogeneous nucleation processes. Hisatome et al. teach in Nephron, 65(4), 1993, 578-582 that alkalinisation of urine by sodium or potassium citrate may be an effective treatment for uric acid stones. Grases et al. describe in Nutr. J., 5(1), 2006, 23-29 the prevention of uric acid calculi by increasing the pH of urine alkalinising agents such as citrate or bicarbonate. Heilberg et al. disclose in Adv. Chron. Kidney Dis., 20(2), 2013, 165-174 that the most important variable in the causation of urinary stones is low urine pH. The mainstay of therapy is urine alkalinisation. Ingestion of vegetables high in citrate increases urine pH and is associated with fewer stones. Grases et al. disclose in Nephron, 73(4), 1996, 561-568 that citrate totally inhibits the development of calcium oxalate monohydrate (COM) crystals. Saponins such as aescin and glycyrrhizic acid exhibit important inhibitory effects on COM crystallisation but do not completely prevent formation of crystals.

This generates the need to be able to increase the pH of urine in an effective, controlled manner, in order to prevent uric acid oversaturation and, as such, lithiasis.

### DESCRIPTION OF THE INVENTION

The present invention aims to find new formulae for treating uric acid renal lithiasis or calcium oxalate renal lithiasis induced by uric acid. This combination proves highly beneficial for treating uric acid renal calculi or calcium oxalate renal calculi induced by uric acid, since the beneficial effect of uric acid crystallization inhibitors is added to increased urinary pH values , which together, make it possible to achieve even greater beneficial effects with a lower dose of basifying agent, thus preventing the risk of excessively high urinary pH values being reached, and consequently, the risk of inducing the formation of phosphate lithiasis.

A first aspect of the present invention therefore relates to a preparation comprising at least one urinary basifying agent combined with at least one uric acid crystallization inhibitor for use sequentially, simultaneously or separately in the treatment or prevention of renal lithiasis; and wherein the urinary basifying agent is selected from potassium citrate, magnesium citrate, sodium bicarbonate, potassium bicarbonate, magnesium bicarbonate or mixtures thereof; and wherein the crystallization inhibitor is selected from saponins.

The term "urinary basifying agent" refers to all those substances currently known about, which when administered orally, increase urinary pH values. At present, some of the most well-known and frequently used examples include potassium citrate or mixtures of potassium and magnesium citrate. The doses used range from 300 mg/day to 6000 mg/day. Mixtures of sodium bicarbonate and potassium bicarbonate have also been used as basifying agents, in doses ranging from 45-105 mM/day or 60-120 mM/day.
The term "renal lithiasis", "urolithiasis" or "nephrolithiasis" refers to the disorder caused by the presence of calculi or stones found in the kidneys or urinary tract (ureters, bladder). Renal calculi are made up of substances usually found in urine (calcium salts, uric acid, cystine etc.), which, for various reasons, have been concentrated and precipitated, forming fragments of varying size.
The term "uric acid crystals" or "uric acid calculi" includes all those processes or conditions that give rise to or induce the formation of solid precipitates in urine, in which this substance is involved.

According to the invention, the urinary basifying agent is selected from potassium citrate, magnesium citrate, sodium bicarbonate, potassium bicarbonate, magnesium bicarbonate or mixtures thereof.

Other polyhydroxycarboxylic acids constitute basifying agents, examples of which include ethylenediaminetetraacetic acid, isocitric acid, malic acid and succinic acid.

According to the invention, the crystallization inhibitor is selected from saponins.

In a more preferred embodiment, saponins are derived from part of a plant or animal species rich in these compounds, or a plant or animal extract taken from said species rich in these compounds.
In another preferred embodiment, the medicament is presented in a form suitable for oral, parenteral, intravenous and enteral administration.
In another preferred embodiment, the renal lithiasis is an oxalocalcic and induced by uric acid.

In another preferred embodiment, the renal lithiasis is mixed and induced by uric acid and calcium oxalate.

In another embodiment of the invention, the preparation for use in the treatment or prevention of renal lithiasis is a composition comprising at least one urinary basifying agent and at least one uric acid crystallization inhibitor, as defined in the claims.

According to the invention, the urinary basifying agent is selected from potassium citrate, magnesium citrate, sodium bicarbonate, potassium bicarbonate, magnesium bicarbonate or mixtures thereof.

According to the invention, the crystallization inhibitor is selected from saponins.

In a more preferred embodiment, saponins are derived from part of a plant or animal species rich in these compounds, or a plant or animal extract taken from said species rich in these compounds.
In another preferred embodiment, the composition of the invention comprises between 25- 45 % by weight of the urinary basifying agent and between 20-30 % by weight of the crystallization inhibitor. The following constitute examples of the composition for use in the invention:

**Composition 1.**

| Compound | Amount |
|---|---|
| Potassium citrate | 300 mg |
| Magnesium citrate | 250 mg |
| Ginseng extract rich saponins > 30 % | 150 mg |

**Composition 2.**

| Compound | Amount |
|---|---|
| Potassium citrate | 350 mg |
| Magnesium citrate | 200 mg |
| Glycyrrhizic acid | 160 mg |

**Composition 3.**

| Compound | Amount |
|---|---|
| Sodium bicarbonate | 4.20 mg |
| Potassium bicarbonate | 5.10 mg |
| Indian chestnut seed extract equivalent to the following amount of Aescin | 50 mg |

**Composition 4.**

| Compound | Amount |
|---|---|
| Sodium bicarbonate | 6 mg |
| Potassium bicarbonate | 7 mg |
| Aescin | 60 mg |

In a preferred embodiment, the composition is a pharmaceutical composition or a nutraceutical or functional food.

In the present invention, the term "nutraceutical" or "functional food" is understood to refer to a food that has a beneficial effect on health. Similarly, the term "nutraceutical" may be applied to chemical extracts or compounds derived from common foods. Examples of foods to which nutraceutical properties are attributed include olive oil, red wine, broccoli and soya, etc. Nutraceuticals are usually employed in nutritional mixtures and in the pharmaceutical industry. Like various foodstuffs, some nutritional supplements may also be classified as nutraceuticals, for example fatty acids such as omega-3 derived from fish oils and from some vegetables or antioxidants and vitamins.

The combination of urinary basifying agents and uric acid crystallization inhibitors may be administered in solid form (including granules, powder or suppositories) or in liquid form (as solutions, suspensions or emulsions). In turn, they may be administered as such or, after being subjected to operations such as sterilization, the addition of preservatives, the addition of stabilizers or the addition of emulsifiers.
The co-administration of urinary basifying agents and uric acid crystallization inhibitors may be combined with one or more compounds that facilitate the absorption thereof, via the selected administrative route. As such, they may be administered with lactose, sucrose, talc, magnesium stearate, cellulose, calcium salts, gelatin and fatty acids, as well as other similar substances.
The pharmaceutically acceptable adjuvants and vehicles that may be utilized in said compositions constitute those adjuvants and vehicles known about by ordinary experts skilled in the art, which are commonly used to prepare therapeutic compositions.
In order to be used for therapeutic purposes, the urinary basifying agents and uric acid crystallization inhibitors will preferably be found, in a pharmaceutically acceptable or substantially pure form, i.e., with an acceptable pharmaceutical purity level, excluding normal pharmaceutical additives such as diluents and carriers, and not including material considered toxic at normal dosage levels. The purity levels for the active ingredient are preferably higher than 50 %, more preferably higher than 70 % and more preferably still, higher than 90 %. In a preferred embodiment, they are higher than 95 % of the formula (I) compound or the salts or solvates thereof.

Throughout the description and claims, the word "comprises" and variations thereof are not intended to exclude another technical characteristics, additives, components or steps. The following example and drawings are provided by way of illustration of the present invention.

### BRIEF DESCRIPTION OF THE DRAWINGS

**FIG. 1****.** Is a graph showing the induction times (in minutes) for a uric acid solution at 400 mg/L in phosphate buffer, at different pH values and aescin concentrations.
**FIG. 2****.** Is a graph showing the induction times (in minutes) for a uric acid solution at 400 mg/L in phosphate buffer, at different pH values and glycyrrhizic acid concentrations.

### EXAMPLE

### Example 1: measuring uric acid crystallization in the presence of saponins

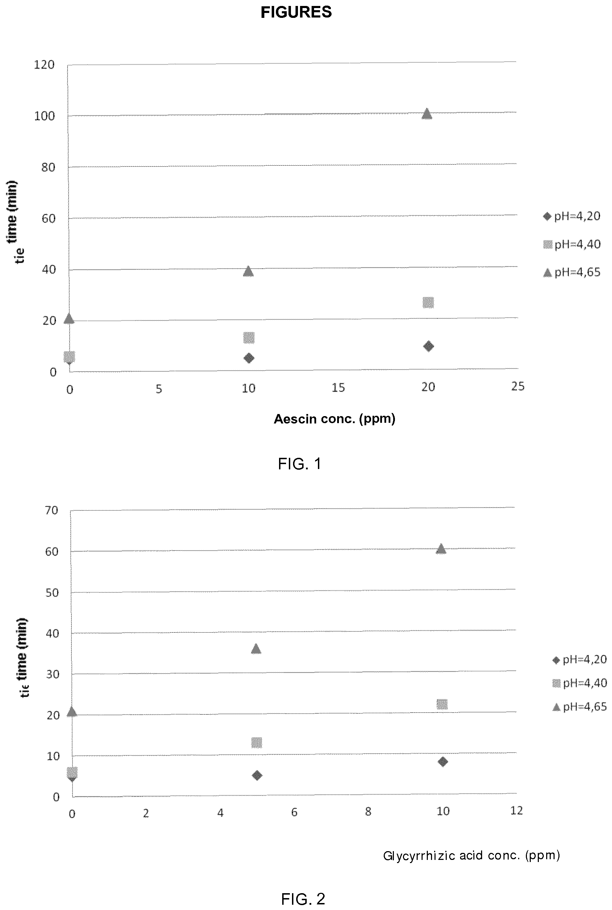

Induction times for uric acid crystallization at different pH values and aescin concentrations were calculated from a 400 mg/L solution of uric acid in a phosphate buffer, with an ionic strength similar to that of urine. In Table 1, it is possible to observe these induction times, which, as can be clearly observed (Figure 1), increase when the pH and aescin concentration values are in turn increased. As such, whilst at pH 4.2, a uric acid solution (without an inhibitor) takes 5 minutes to crystallize, with 20 ppm of aescin, it takes 9 minutes (there is a 4 minute delay in the induction time at this pH value, for 20 ppm of aescin). However, upon increasing the pH to 4.4, uric acid (without an inhibitor) takes 6 minutes to crystallize, whilst with 20 ppm of aescin, the same solution takes 26 minutes to crystallize (there is a 20 minute delay in the induction time at this pH value, for 20 ppm of aescin). For an equal amount of inhibitor, a significant increase in the crystallization time is thus produced upon increasing the pH value of the solution.

**Table 1: Induction times (in minutes) for a 400 mg/L uric acid solution in phosphate buffer, at different pH values and aescin concentrations.**

| | **t (min) pH=4.20** | **t (min) pH=4.40** | **t (min) pH=4.65** |
|---|---|---|---|
| 0 ppm of aescin | 5 | 6 | 21 |
| 10 ppm of aescin | 5 | 13 | 39 |
| 20 ppm of aescin | 9 | 26 | 100 |

The same behavior is evidenced for another uric acid crystallization inhibitor, namely glycyrrhizic acid, as can be seen looking at the results in table 2 and figure 2 below.

**Table 2: Induction times (in minutes) for a 400 mg/L solution of uric acid in phosphate buffer, at different pH values and glycyrrhizic acid concentrations.**

| | **t (min) pH=4.20** | **t (min) pH=4.40** | **t (min) pH=4.65** |
|---|---|---|---|
| 0 ppm of Glyc. ac. | 5 | 6 | 21 |
| 5 ppm of Glyc. ac. | 5 | 13 | 36 |
| 10 ppm of Glyc. ac. | 8 | 22 | 60 |

It is possible to conclude that, as shown in the experimental evidence presented, for an equal amount of inhibitor, a substantial increase in its inhibitory capacity is produced upon increasing the pH of the medium, a fact that was not known until now, which demonstrates a synergistic effect between urinary basification and crystallization inhibition.

## Claims

1. A preparation comprising at least one urinary basifying agent combined with at least one uric acid crystallization inhibitor for use sequentially, simultaneously or separately in the treatment or prevention of renal lithiasis; and
wherein the urinary basifying agent is selected from potassium citrate, magnesium citrate, sodium bicarbonate, potassium bicarbonate, magnesium bicarbonate or mixtures thereof; and
wherein the crystallization inhibitor is selected from saponins.

2. The preparation for use according to claim 1, wherein the saponins are derived from part of a plant or animal species rich in these compounds, or a plant or animal extract taken from said species rich in these compounds.

3. The preparation for use according to any of the previous claims, which is a medicament in a form suitable for oral, parenteral, intravenous or enteral administration.

4. The preparation for use according to any of the claims 1 to 3, wherein the renal lithiasis is oxalocalcic and induced by uric acid.

5. The preparation for use according to any of the claims 1 to 4, wherein the renal lithiasis is mixed and induced by uric acid and calcium oxalate.

6. The preparation for use according to any of the previous claims, wherein the preparation is a composition comprising at least one urinary basifying agent according to any of the previous claims and at least one uric acid crystallization inhibitor according to any of the previous claims.

7. The preparation for use according to claim 6 wherein the composition is a pharmaceutical composition or a nutraceutical or functional food.

8. The preparation for use according to any of claims 6 and 7, wherein the composition comprises between 25- 45 % by weight of the urinary basifying agent and between 20-30 % by weight of the crystallization inhibitor.

## Patentansprüche

1. Ein pharmazeutisches Präparat umfassend mindestens ein den Harn betreffendes alkalisierendes Mittel in Kombination mit mindestens einem Harnsäurekristallisationshemmer zur aufeinander folgenden, gleichzeitigen oder voneinander getrennten Verwendung bei der Behandlung oder Prävention von Nierensteinbildung; und
wobei das den Harn betreffende alkalisierende Mittel ausgewählt aus Kaliumcitrat, Magnesiumcitrat, Natriumhydrogencarbonat, Kaliumhydrogencarbonat, Magnesiumhydrogencarbonat oder Mischungen davon ist; und
wobei der Kristallisationshemmer aus Saponinen ausgewählt ist.

2. Das pharmazeutische Präparat zur Verwendung nach Anspruch 1, wobei die Saponine von einem Teil einer Pflanzen- oder einer Tierart, die reich an diesen Verbindungen ist, oder einem aus den an diesen Verbindungen reichen Arten gewonnenen Pflanzen- oder Tierextrakt, abgeleitet sind.

3. Das pharmazeutische Präparat zur Verwendung nach einem der vorhergehenden Ansprüche, welches ein Arzneimittel in einer zur oralen, parenteralen, intravenösen oder enteralen Verabreichung geeigneten Form ist.

4. Das pharmazeutische Präparat zur Verwendung nach einem der Ansprüche 1 bis 3, wobei die Nierensteinbildung auf Oxalat-Calcium beruht und durch Harnsäure herbeigeführt wird.

5. Das pharmazeutische Präparat zur Verwendung nach einem der Ansprüche 1 bis 4, wobei die Nierensteinbildung gemischt ist und durch Harnsäure und Calciumoxalat herbeigeführt wird.

6. Das pharmazeutische Präparat zur Verwendung nach einem der vorhergehenden Ansprüche, wobei das pharmazeutische Präparat eine Zusammensetzung umfassend mindestens ein den Harn betreffendes alkalisierendes Mittel nach einem der vorhergehenden Ansprüche und mindestens einen Harnsäurekristallisationshemmer nach einem der vorhergehenden Ansprüche ist.

7. Das pharmazeutische Präparat zur Verwendung nach Anspruch 6, wobei die Zusammensetzung eine pharmazeutische Zusammensetzung oder ein Neutraceutical oder funktionelles Lebensmittel ist.

8. Das pharmazeutische Präparat zur Verwendung nach einem der Ansprüche 6 und 7, wobei die Zusammensetzung zwischen 25-45 Gew.-% des den Harn betreffenden alkalisierenden Mittels und zwischen 20-30 Gew.-% des Kristallisationshemmers umfasst.

## Revendications

1. Une préparation comprenant au moins un agent alcalinisant urinaire combiné avec au moins un inhibiteur de la cristallisation de l'acide urique pour l'utilisation séquentielle, de façon simultanée ou séparée dans le traitement ou la prévention de la lithiase rénale ; et
dans laquelle l'agent alcalinisant urinaire est choisi parmi le citrate de potassium, le citrate de magnésium, le bicarbonate de sodium, le bicarbonate de potassium, le bicarbonate de magnésium ou des mélanges de ceux-ci ; et
dans laquelle l'inhibiteur de la cristallisation est choisi parmi les saponines.

2. La préparation pour l'utilisation selon la revendication 1, dans laquelle les saponines sont dérivées d'une partie d'une espèce végétale ou animale riche en ces composés, ou d'un extrait végétal ou animal pris de ladite espèce riche en ces composés.

3. La préparation pour l'utilisation selon l'une quelconque des revendications précédentes, qui est un médicament dans une forme appropriée pour l'administration orale, parentérale, intraveineuse ou entérale.

4. La préparation pour l'utilisation selon l'une quelconque des revendications 1 à 3, dans laquelle la lithiase rénale est de nature oxalo-calcique et induite par l'acide urique.

5. La préparation pour l'utilisation selon l'une quelconque des revendications 1 à 4, dans laquelle la lithiase rénale est de nature mixte et induite par l'acide urique et l'oxalate de calcium.

6. La préparation pour l'utilisation selon l'une quelconque des revendications précédentes, dans laquelle la préparation est une composition comprenant au moins un agent alcalinisant urinaire selon l'une quelconque des revendications précédentes et au moins un inhibiteur de la cristallisation de l'acide urique selon l'une quelconque des revendications précédentes.

7. La préparation pour l'utilisation selon la revendication 6, dans laquelle la composition est une composition pharmaceutique ou un nutraceutique ou alicament.

8. La préparation pour l'utilisation selon l'une quelconque des revendications 6 et 7, dans laquelle la composition comprend entre 25-45 % en poids de l'agent alcalinisant urinaire et entre 20-30% en poids de l'inhibiteur de la cristallisation.
